# EUROPEAN PATENT APPLICATION

(11) **EP 1 586 374 A2**
(43) Date of publication of application: **19.10.2005**
(21) Application number: 05251938.6
(22) Date of filing: 29.03.2005
(51) Int. Cl.: B01J 19/00

(54) **Methods for in situ generation of nucleic acid arrays**

(30) Priority: 29.03.2004 US 813337
(71) Applicant: Agilent Technologies, Inc., Palo Alto, CA 94306 (US)
(72) Inventor: Peck, Bill, J., Mountain View, CA 94043 (US); Leproust, Eric, M., San Jose, CA 95125 (US)
(74) Representative: Tollett, Ian

(57) **Abstract**

Methods of producing nucleic acid arrays using an *in situ* nucleic acid synthesis protocol are provided, where the *in situ* nucleic acid synthesis protocol includes a plurality of cycles, each of which includes: (I) a monomer attachment step; and (II) a functional group generation step. A feature of the subject methods is that the functional group generation step is performed by sequentially flowing a plurality of different fluids across the surface of a substrate (110) in a manner such that the surface is not contacted with a with a triple phase interface line. Also provided are the arrays (112) produced using the subject methods, as well as methods for use of the arrays and kits that include the same.

## Description

The present invention relates to a method of producing an array, apparatus for synthesizing an array and to software for controlling said apparatus.

Arrays of nucleic acids have become an increasingly important tool in the biotechnology industry and related fields. These nucleic acid arrays, in which a plurality of distinct or different nucleic acids are positioned on a solid support surface in the form of an array or pattern, find use in a variety of applications, including gene expression analysis, drug screening, nucleic acid sequencing, mutation analysis, and the like.

A feature of many arrays that have been developed is that each of the distinct nucleic acids of the array is stably attached to a discrete location on the array surface, such that its position remains constant and known throughout the use of the array. Stable attachment is achieved in a number of different ways, including covalent bonding of the polymer to the support surface and noncovalent interaction of the polymer with the surface.

There are two main ways of producing nucleic acid arrays in which the immobilized nucleic acids are covalently attached to the substrate surface, i.e., via *in situ* synthesis in which the nucleic acid ligand is grown on the surface of the substrate in a step-wise fashion and via deposition of the full ligand, e.g., a presynthesized nucleic acid/polypeptide, cDNA fragment, etc., onto the surface of the array.

Where the *in situ* synthesis approach is employed, conventional phosphoramidite synthesis protocols are typically used. In phosphoramidite synthesis protocols, the 3'-hydroxyl group of an initial 5'-protected nucleoside is first covalently attached to the polymer support, e.g., a planar substrate surface. Synthesis of the nucleic acid then proceeds by deprotection of the 5'-hydroxyl group of the attached nucleoside, followed by coupling of an incoming nucleoside-3'-phosphoramidite to the deprotected 5' hydroxyl group (5'-OH). The resulting phosphite triester is finally oxidized to a phosphotriester to complete the internucleotide bond. The steps of deprotection, coupling and oxidation are repeated until a nucleic acid of the desired length and sequence is obtained. Optionally, a capping reaction may be used after the coupling and/or after the oxidation to inactivate the growing DNA chains that failed in the previous coupling step, thereby avoiding the synthesis of inaccurate sequences.

The chemical group conventionally used for the protection of nucleoside 5'-hydroxyls is dimethoxytrityl ("DMT"), which group is removable with acid. This acid-labile protecting group provides a number of advantages for working with both nucleosides and oligonucleotides. For example, the DMT group can be introduced onto a nucleoside regioselectively and in high yield. Also, the lipophilicity of the DMT group greatly increases the solubility of nucleosides in organic solvents, and the carbocation resulting from acidic deprotection gives a strong chromophore, which can be used to indirectly monitor coupling efficiency. In addition, the hydrophobicity of the group can be used to aid separation on reverse-phase HPLC.

However, use of DMT as a hydroxyl-protecting group in nucleic acid synthesis is also problematic. The N-glycosidic linkages of nucleic acids are susceptible to acid catalyzed cleavage, and even when the protocol is optimized, recurrent removal of the DMT group with acid during synthesis results in depurination. The N-6-benzoyl-protected deoxyadenosine nucleotide is especially susceptible to glycosidic cleavage, resulting in a substantially reduced yield of the final nucleic acid. In the context of in situ nucleic acid array synthesis, glycisidic bond cleavage as described above leads to cleavage of the phosphotriester during deprotection, which results in the production of shorter sequences and inaccurate signal intensities.

In the synthesis of nucleic acids on the surface of a nucleic acid array, reactive deoxynucleoside phosphoramidites are successively applied, in molecular amounts exceeding the molecular amounts of target hydroxyl groups of the substrate or growing oligonucleotide polymers, to specific cells of the high-density array, where they chemically bond to the target hydroxyl groups. Then, unreacted deoxynucleoside phosphoramidites from multiple cells of the high-density array are washed away, oxidation of the phosphite bonds joining the newly added deoxynucleosides to the growing oligonucleotide polymers to form phosphate bonds is carried out, and unreacted hydroxyl groups of the substrate or growing oligonucleotide polymers are chemically capped to prevent them from reacting with subsequently applied deoxynucleoside phosphoramidites. Optionally, the capping reaction may be done prior to oxidation.

While nucleic acid arrays have been manufactured using *in situ* synthesis techniques, as described above, the problems associated with the use of DMT are exacerbated in protocols where "microscale" parallel reactions are taking place on a very dense, packed surface, e.g., as occurs in the fabrication of many types of nucleic acid arrays. Applications in the field of genomics and high throughput screening have fueled the demand for precise chemistry in such a context. Thus, increasingly stringent demands are placed on the chemical synthesis cycle as it was originally conceived, and the problems associated with conventional methods for synthesizing oligonucleotides are rising to unacceptable levels in these expanded applications.

Accordingly, there is continued interest in the development of new protocols for producing nucleic acid arrays via *in situ* synthesis. Of particular interest would be the development of a protocol that was amenable to automated applications and resulted in high quality arrays by at least reducing, if not substantially eliminating, depurination side reactions during deblocking.

### Relevant Literature

U.S. Patents of interest include: 6,020,475; 6,222,030; and 6,300,137. See also U.S. Published Patent Application Nos. 20030003222; 20030003504; 20030112022; 200030228422; 200030232123; and 20030232140.

Methods of producing nucleic acid arrays using an *in situ* nucleic acid synthesis protocol are provided, where the *in situ* nucleic acid synthesis protocol includes a plurality of cycles, each of which includes: (I) a monomer attachment step; and (II) a 5' functional group generation step. A feature of the subject methods is that the 5' functional group generation step is performed by sequentially flowing a plurality of different fluids, e.g., oxidizing, deblocking and washing fluids, across the surface of a substrate, e.g., in a flow cell, in a manner such that the surface is not contacted with a triple phase interface line while the solution phase contains an active reagent. Also provided are the arrays produced using the subject methods, as well as methods for use of the arrays and kits that include the same.

A number of preferred embodiments of the present invention will now be described with reference to the drawings, in which:-
Figure 1 shows an exemplary substrate carrying an array, such as may be used in the devices of the subject invention.
Figure 2 shows an enlarged view of a portion of Figure 1 showing spots or features.
Figure 3 is an enlarged view of a portion of the substrate of Figure 1.
Figure 4 is a schematic diagram depicting an embodiment of an apparatus for conducting synthesis of arrays according to a representative embodiment of the subject invention.

Unless defined otherwise, all technical and scientific terms used herein have the same meaning as commonly understood by one of ordinary skill in the art to which this invention belongs. Still, certain elements are defined below for the sake of clarity and ease of reference.

The term "biomolecule" means any organic or biochemical molecule, group or species of interest that may be formed in an array on a substrate surface. Exemplary biomolecules include peptides, proteins, amino acids and nucleic acids.

The term "peptide" as used herein refers to any compound produced by amide formation between a carboxyl group of one amino acid and an amino group of another group.

The term "oligopeptide" as used herein refers to peptides with fewer than about 10 to 20 residues, *i.e*. amino acid monomeric units.

The term "polypeptide" as used herein refers to peptides with more than 10 to 20 residues.

The term "protein" as used herein refers to polypeptides of specific sequence of more than about 50 residues.

The term "nucleic acid" as used herein means a polymer composed of nucleotides, e.g., deoxyribonucleotides or ribonucleotides, or compounds produced synthetically (e.g. PNA as described in U.S. Patent No. 5,948,902 and the references cited therein) which can hybridize with naturally occurring nucleic acids in a sequence specific manner analogous to that of two naturally occurring nucleic acids, e.g., can participate in Watson-Crick base pairing interactions.

The terms "nucleoside" and "nucleotide" are intended to include those moieties that contain not only the known purine and pyrimidine base moieties, but also other heterocyclic base moieties that have been modified. Such modifications include methylated purines or pyrimidines, acylated purines or pyrimidines, or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The terms "ribonucleic acid" and "RNA" as used herein refer to a polymer composed of ribonucleotides.

The terms "deoxyribonucleic acid" and "DNA" as used herein mean a polymer composed of deoxyribonucleotides.

The term "oligonucleotide" as used herein denotes single stranded nucleotide multimers of from about 10 to 100 nucleotides and up to 200 nucleotides in length.

The term "polynucleotide" as used herein refers to single or double stranded polymer composed of nucleotide monomers of generally greater than 100 nucleotides in length.

A "biopolymer" is a polymeric biomolecule of one or more types of repeating units. Biopolymers are typically found in biological systems and particularly include polysaccharides (such as carbohydrates), peptides (which term is used to include polypeptides and proteins) and polynucleotides as well as their analogs such as those compounds composed of or containing amino acid analogs or non-amino acid groups, or nucleotide analogs or non-nucleotide groups.

A "biomonomer" references a single unit, which can be linked with the same or other biomonomers to form a biopolymer (e.g., a single amino acid or nucleotide with two linking groups, one or both of which may have removable protecting groups).

An "array," includes any one-dimensional, two-dimensional or substantially two-dimensional (as well as a three-dimensional) arrangement of addressable regions bearing a particular chemical moiety or moieties (such as ligands, e.g., biopolymers such as polynucleotide or oligonucleotide sequences (nucleic acids), polypeptides (e.g., proteins), carbohydrates, lipids, etc.) associated with that region. In the broadest sense, the arrays of many embodiments are arrays of polymeric binding agents, where the polymeric binding agents may be any of: polypeptides, proteins, nucleic acids, polysaccharides, synthetic mimetics of such biopolymeric binding agents, etc. In many embodiments of interest, the arrays are arrays of nucleic acids, including oligonucleotides, polynucleotides, cDNAs, mRNAs, synthetic mimetics thereof, and the like. Where the arrays are arrays of nucleic acids, the nucleic acids may be covalently attached to the arrays at any point along the nucleic acid chain, but are generally attached at one of their termini (e.g. the 3' or 5' terminus). Sometimes, the arrays are arrays of polypeptides, e.g., proteins or fragments thereof.

Any given substrate may carry one, two, four or more or more arrays disposed on a front surface of the substrate. Depending upon the use, any or all of the arrays may be the same or different from one another and each may contain multiple spots or features. A typical array may contain more than ten, more than one hundred, more than one thousand more ten thousand features, or even more than one hundred thousand features, in an area of less than 20 cm² or even less than 10 cm². For example, features may have widths (that is, diameter, for a round spot) in the range from a 10 µm to 1.0 cm. In other embodiments each feature may have a width in the range of 1.0 µm to 1.0 mm, usually 5.0 µm to 500 µm, and more usually 10 µm to 200 µm. Non-round features may have area ranges equivalent to that of circular features with the foregoing width (diameter) ranges. At least some, or all, of the features are of different compositions (for example, when any repeats of each feature composition are excluded the remaining features may account for at least 5%, 10%, or 20% of the total number of features). Interfeature areas will typically (but not essentially) be present which do not carry any polynucleotide (or other biopolymer or chemical moiety of a type of which the features are composed). Such interfeature areas typically will be present where the arrays are formed by processes involving drop deposition of reagents but may not be present when, for example, light directed synthesis fabrication processes are used. It will be appreciated though, that the interfeature areas, when present, could be of various sizes and configurations.

Each array may cover an area of less than 100 cm², or even less than 50 cm², 10 cm² or 1 cm². In many embodiments, the substrate carrying the one or more arrays will be shaped generally as a rectangular solid (although other shapes are possible), having a length of more than 4 mm and less than 1 m, usually more than 4 mm and less than 600 mm, more usually less than 400 mm; a width of more than 4 mm and less than 1 m, usually less than 500 mm and more usually less than 400 mm; and a thickness of more than 0.01 mm and less than 5.0 mm, usually more than 0.1 mm and less than 2 mm and more usually more than 0.2 and less than 1 mm. With arrays that are read by detecting fluorescence, the substrate may be of a material that emits low fluorescence upon illumination with the excitation light. Additionally in this situation, the substrate may be relatively transparent to reduce the absorption of the incident illuminating laser light and subsequent heating if the focused laser beam travels too slowly over a region. For example, substrate 10 may transmit at least 20%, or 50% (or even at least 70%, 90%, or 95%), of the illuminating light incident on the front as may be measured across the entire integrated spectrum of such illuminating light or alternatively at 532 nm or 633 nm.

Arrays can be fabricated using drop deposition from pulsejets of either polynucleotide precursor units (such as monomers) in the case of *in situ* fabrication, or the previously obtained polynucleotide. Such methods are described in detail in, for example, the previously cited references including US 6,242,266, US 6,232,072, US 6,180,351, US 6,171,797, US 6,323,043, U.S. Patent Application Serial No. 09/302,898 filed April 30, 1999 by Caren et al., and the references cited therein. These references are incorporated herein by reference. Other drop deposition methods can be used for fabrication, as previously described herein.

The term "monomer" as used herein refers to a chemical entity that can be covalently linked to one or more other such entities to form an polymer. Of particular interest to the present application are nucleotide "monomers" that have first and second sites (e.g., 5' and 3' sites) suitable for binding to other like monomers by means of standard chemical reactions (e.g., nucleophilic substitution), and a diverse element which distinguishes a particular monomer from a different monomer of the same type (e.g., a nucleotide base, etc.). In the art synthesis of nucleic acids of this type utilizes an initial substrate-bound monomer that is generally used as a building-block in a multi-step synthesis procedure to form a complete nucleic acid.

The term "oligomer" is used herein to indicate a chemical entity that contains a plurality of monomers. As used herein, the terms "oligomer" and "polymer" are used interchangeably, as it is generally, although not necessarily, smaller "polymers" that are prepared using the functionalized substrates of the invention, particularly in conjunction with combinatorial chemistry techniques. Examples of oligomers and polymers include polydeoxyribonucleotides (DNA), polyribonucleotides (RNA), other polynucleotides which are C-glycosides of a purine or pyrimidine base. In the practice of the instant invention, oligomers will generally comprise about 2-60 monomers, preferably about 10-60, more preferably about 50-60 monomers.

The term "sample" as used herein relates to a material or mixture of materials, typically, although not necessarily, in fluid form, containing one or more components of interest.

The terms "nucleoside" and "nucleotide" are intended to include those moieties which contain not only the known purine and pyrimidine bases, but also other heterocyclic bases that have been modified. Such modifications include methylated purines or pyrimidines, acylated purines or pyrimidines, alkylated riboses or other heterocycles. In addition, the terms "nucleoside" and "nucleotide" include those moieties that contain not only conventional ribose and deoxyribose sugars, but other sugars as well. Modified nucleosides or nucleotides also include modifications on the sugar moiety, e.g., wherein one or more of the hydroxyl groups are replaced with halogen atoms or aliphatic groups, or are functionalized as ethers, amines, or the like.

The terms "protection" and "deprotection" as used herein relate, respectively, to the addition and removal of chemical protecting groups using conventional materials and techniques within the skill of the art and/or described in the pertinent literature; for example, reference may be had to Greene et al., Protective Groups in Organic Synthesis, 2nd Ed., New York: John Wiley & Sons, 1991. Protecting groups prevent the site to which they are attached from participating in the chemical reaction to be carried out.

"Optional" or "optionally" means that the subsequently described circumstance may or may not occur, so that the description includes instances where the circumstance occurs and instances where it does not. For example, the phrase "optionally substituted" means that a non-hydrogen substituent may or may not be present, and, thus, the description includes structures wherein a non-hydrogen substituent is present and structures wherein a non-hydrogen substituent is not present.

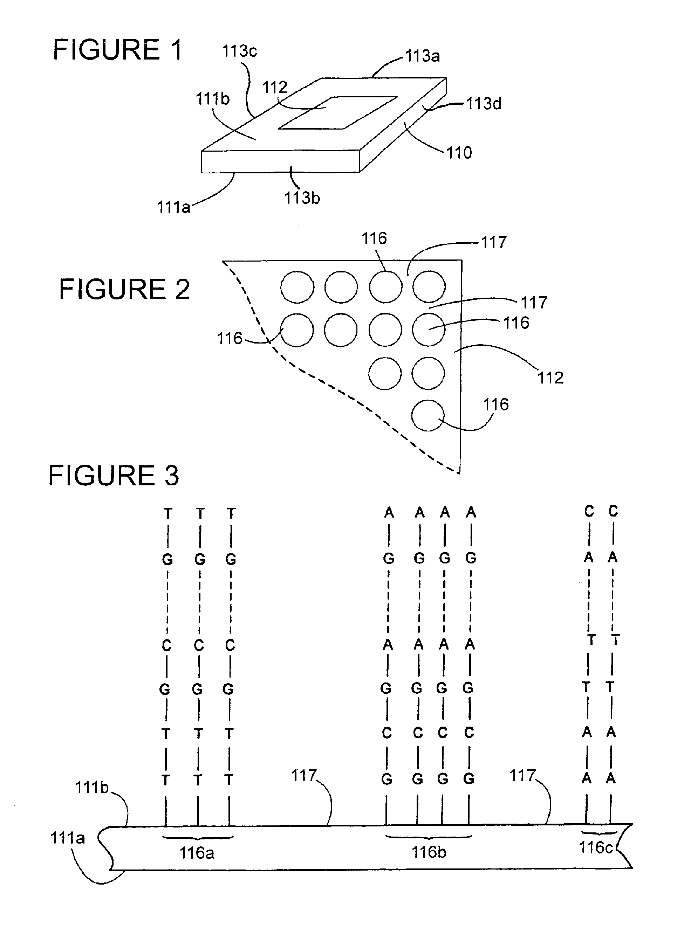

An exemplary array is shown in Figures 1-3, where the array shown in this representative embodiment includes a contiguous planar substrate 110 carrying an array 112 disposed on a rear surface 111 b of substrate 110. It will be appreciated though, that more than one array (any of which are the same or different) may be present on rear surface 111 b, with or without spacing between such arrays. That is, any given substrate may carry one, two, four or more arrays disposed on a front surface of the substrate and depending on the use of the array, any or all of the arrays may be the same or different from one another and each may contain multiple spots or features. The one or more arrays 112 usually cover only a portion of the rear surface 111 b, with regions of the rear surface 111 b adjacent the opposed sides 113c, 113d and leading end 113a and trailing end 113b of slide 110, not being covered by any array 112. A front surface 111a of the slide 110 does not carry any arrays 112. Each array 112 can be designed for testing against any type of sample, whether a trial sample, reference sample, a combination of them, or a known mixture of biopolymers such as polynucleotides. Substrate 110 may be of any shape, as mentioned above.

As mentioned above, array 112 contains multiple spots or features 116 of biopolymers, e.g., in the form of polynucleotides. As mentioned above, all of the features 116 may be different, or some or all could be the same. The interfeature areas 117 could be of various sizes and configurations. Each feature carries a predetermined biopolymer such as a predetermined polynucleotide (which includes the possibility of mixtures of polynucleotides). It will be understood that there may be a linker molecule (not shown) of any known types between the rear surface 111 b and the first nucleotide.

Substrate 110 may carry on front surface 111 a, an identification code, e.g., in the form of bar code (not shown) or the like printed on a substrate in the form of a paper label attached by adhesive or any convenient means. The identification code contains information relating to array 112, where such information may include, but is not limited to, an identification of array 112, i.e., layout information relating to the array(s), etc.

In those embodiments where an array includes two more features immobilized on the same surface of a solid support, the array may be referred to as addressable. An array is "addressable" when it has multiple regions of different moieties (e.g., different polynucleotide sequences) such that a region (i.e., a "feature" or "spot" of the array) at a particular predetermined location (i.e., an "address") on the array will detect a particular target or class of targets (although a feature may incidentally detect non-targets of that feature). Array features are typically, but need not be, separated by intervening spaces. In the case of an array, the "target" will be referenced as a moiety in a mobile phase (typically fluid), to be detected by probes ("target probes") which are bound to the substrate at the various regions. However, either of the "target" or "probe" may be the one which is to be evaluated by the other (thus, either one could be an unknown mixture of analytes, e.g., polynucleotides, to be evaluated by binding with the other).

A "scan region" refers to a contiguous (preferably, rectangular) area in which the array spots or features of interest, as defined above, are found. The scan region is that portion of the total area illuminated from which the resulting fluorescence is detected and recorded. For the purposes of this invention, the scan region includes the entire area of the slide scanned in each pass of the lens, between the first feature of interest, and the last feature of interest, even if there exist intervening areas which lack features of interest. An "array layout" refers to one or more characteristics of the features, such as feature positioning on the substrate, one or more feature dimensions, and an indication of a moiety at a given location. "Hybridizing" and "binding", with respect to polynucleotides, are used interchangeably.

The term "substrate" as used herein refers to a surface upon which marker molecules or probes, e.g., an array, may be adhered. Glass slides are the most common substrate for biochips, although fused silica, silicon, plastic and other materials are also suitable.

The term "flexible" is used herein to refer to a structure, e.g., a bottom surface or a cover, that is capable of being bent, folded or similarly manipulated without breakage. For example, a cover is flexible if it is capable of being peeled away from the bottom surface without breakage.

"Flexible" with reference to a substrate or substrate web, references that the substrate can be bent 180 degrees around a roller of less than 1.25 cm in radius. The substrate can be so bent and straightened repeatedly in either direction at least 100 times without failure (for example, cracking) or plastic deformation. This bending must be within the elastic limits of the material. The foregoing test for flexibility is performed at a temperature of 20 °C.

A "web" references a long continuous piece of substrate material having a length greater than a width. For example, the web length to width ratio may be at least 5/1, 10/1, 50/1, 100/1, 200/1, or 500/1, or even at least 1000/1.

The substrate may be flexible (such as a flexible web). When the substrate is flexible, it may be of various lengths including at least 1 m, at least 2 m, or at least 5 m (or even at least 10 m).

The term "rigid" is used herein to refer to a structure, e.g., a bottom surface or a cover that does not readily bend without breakage, i.e., the structure is not flexible.

The terms "hybridizing specifically to" and "specific hybridization" and "selectively hybridize to," as used herein refer to the binding, duplexing, or hybridizing of a nucleic acid molecule preferentially to a particular nucleotide sequence under stringent conditions.

The term "stringent assay conditions" as used herein refers to conditions that are compatible to produce binding pairs of nucleic acids, e.g., surface bound and solution phase nucleic acids, of sufficient complementarity to provide for the desired level of specificity in the assay while being less compatible to the formation of binding pairs between binding members of insufficient complementarity to provide for the desired specificity. Stringent assay conditions are the summation or combination (totality) of both hybridization and wash conditions.

A "stringent hybridization" and "stringent hybridization wash conditions" in the context of nucleic acid hybridization (e.g., as in array, Southern or Northern hybridizations) are sequence dependent, and are different under different experimental parameters. Stringent hybridization conditions that can be used to identify nucleic acids within the scope of the invention can include, e.g., hybridization in a buffer comprising 50% formamide, 5xSSC, and 1% SDS at 42°C, or hybridization in a buffer comprising 5xSSC and 1% SDS at 65°C, both with a wash of 0.2xSSC and 0. 1% SDS at 65°C. Exemplary stringent hybridization conditions can also include a hybridization in a buffer of 40% formamide, 1 M NaCl, and 1 % SDS at 37°C, and a wash in 1 xSSC at 45°C. Alternatively, hybridization to filter-bound DNA in 0.5 M NaHPO₄, 7% sodium dodecyl sulfate (SDS), 1 mM EDTA at 65°C, and washing in 0.1×SSC/0.1 % SDS at 68°C can be employed. Yet additional stringent hybridization conditions include hybridization at 60°C or higher and 3 × SSC (450 mM sodium chloride/45 mM sodium citrate) or incubation at 42°C in a solution containing 30% formamide, 1 M NaCl, 0.5% sodium sarcosine, 50 mM MES, pH 6.5. Those of ordinary skill will readily recognize that alternative but comparable hybridization and wash conditions can be utilized to provide conditions of similar stringency.

In certain embodiments, the stringency of the wash conditions that set forth the conditions which determine whether a nucleic acid is specifically hybridized to a surface bound nucleic acid. Wash conditions used to identify nucleic acids may include, e.g.: a salt concentration of about 0.02 molar at pH 7 and a temperature of at least about 50°C or about 55°C to about 60°C; or, a salt concentration of about 0.15 M NaCl at 72°C for about 15 minutes; or, a salt concentration of about 0.2xSSC at a temperature of at least about 50°C or about 55°C to about 60°C for about 15 to about 20 minutes; or, the hybridization complex is washed twice with a solution with a salt concentration of about 2xSSC containing 0.1 % SDS at room temperature for 15 minutes and then washed twice by 0.1×SSC containing 0.1% SDS at 68°C for 15 minutes; or, equivalent conditions. Stringent conditions for washing can also be, e.g., 0.2×SSC/0.1 % SDS at 42°C.

A specific example of stringent assay conditions is rotating hybridization at 65°C in a salt based hybridization buffer with a total monovalent cation concentration of 1.5 M (e.g., as described in U.S. Patent Application No. 09/655,482 filed on September 5, 2000, the disclosure of which is herein incorporated by reference) followed by washes of 0.5X SSC and 0.1X SSC at room temperature.

Stringent assay conditions are hybridization conditions that are at least as stringent as the above representative conditions, where a given set of conditions are considered to be at least as stringent if substantially no additional binding complexes that lack sufficient complementarity to provide for the desired specificity are produced in the given set of conditions as compared to the above specific conditions, where by "substantially no more" is meant less than about 5-fold more, typically less than about 3-fold more. Other stringent hybridization conditions are known in the art and may also be employed, as appropriate.

By "remote location," it is meant a location other than the location at which the array is present and hybridization occurs. For example, a remote location could be another location (e.g., office, lab, etc.) in the same city, another location in a different city, another location in a different state, another location in a different country, etc. As such, when one item is indicated as being "remote" from another, what is meant is that the two items are at least in different rooms or different buildings, and may be at least one mile, ten miles, or at least one hundred miles apart. "Communicating" information references transmitting the data representing that information as electrical signals over a suitable communication channel (e.g., a private or public network). "Forwarding" an item refers to any means of getting that item from one location to the next, whether by physically transporting that item or otherwise (where that is possible) and includes, at least in the case of data, physically transporting a medium carrying the data or communicating the data. An array "package" may be the array plus only a substrate on which the array is deposited, although the package may include other features (such as a housing with a chamber). A "chamber" references an enclosed volume (although a chamber may be accessible through one or more ports). It will also be appreciated that throughout the present application, that words such as "top," "upper," and "lower" are used in a relative sense only.

A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

To "record" data, programming or other information on a computer readable medium refers to a process for storing information, using any such methods as known in the art. Any convenient data storage structure may be chosen, based on the means used to access the stored information. A variety of data processor programs and formats can be used for storage, e.g. word processing text file, database format, etc.

A "processor" references any hardware and/or software combination that will perform the functions required of it. For example, any processor herein may be a programmable digital microprocessor such as available in the form of a electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

The present methods and apparatus, as described more fully below, may be used in the synthesis of polypeptides. The synthesis of polypeptides involves the sequential addition of amino acids to a growing peptide chain. This approach comprises attaching an amino acid to the functionalized surface of the support. In one approach the synthesis involves sequential addition of carboxyl-protected amino acids to a growing peptide chain with each additional amino acid in the sequence similarly protected and coupled to the terminal amino acid of the oligopeptide under conditions suitable for forming an amide linkage. Such conditions are well known to the skilled artisan. See, for example, Merrifield, B. (1986), Solid Phase Synthesis, Sciences 232, 341-347. After polypeptide synthesis is complete, acid is used to remove the remaining terminal protecting groups. In accordance with the present invention each of certain repetitive steps involved in the addition of an amino acid may be carried out in a flow cell. Such repetitive steps may involve, among others, washing of the surface, protection and deprotection of certain functionalities on the surface, oxidation or reduction of functionalities on the surface, and so forth.

The apparatus and methods of the present invention are particularly useful in the synthesis of nucleic acid, e.g., oligonucleotide, arrays for determinations of polynucleotides. The synthesis of arrays of polynucleotides on the surface of a support in certain approaches, e.g., in situ fabrication protocols, involves attaching an initial nucleoside or nucleotide to a functionalized surface. In one approach the surface is reacted with nucleosides or nucleotides that are also functionalized for reaction with the groups on the surface of the support. Methods for introducing appropriate amine specific or alcohol specific reactive functional groups into a nucleoside or nucleotide include, by way of example, addition of a spacer amine containing phosphoramidites, addition on the base of alkynes or alkenes using palladium mediated coupling, addition of spacer amine containing activated carbonyl esters, addition of boron conjugates, formation of Schiff bases.

After the introduction of the nucleoside or nucleotide onto the surface, the attached nucleotide may be used to construct the polynucleotide by means well known in the art. For example, in the synthesis of arrays of oligonucleotides, nucleoside monomers are generally employed. In this embodiment an array of the above compounds is attached to the surface and each compound is reacted to attach a nucleoside. Nucleoside monomers are used to form the polynucleotides usually by phosphate coupling, either direct phosphate coupling or coupling using a phosphate precursor such as a phosphite coupling. Such coupling thus includes the use of amidite (phosphoramidite), phosphodiester, phosphotriester, H-phosphonate, phosphite halide, and the like coupling.

One exemplary coupling method is phosphoramidite coupling, which is a phosphite coupling. In using this coupling method, after the phosphite coupling is complete, the resulting phosphite is oxidized to a phosphate. Oxidation can be effected with iodine to give phosphates or with sulfur to give phosphorothioates. The phosphoramidites are dissolved in anhydrous acetonitrile to give a solution having a given ratio of amidite concentrations. The mixture of known chemically compatible monomers is reacted to a solid support, or further along, may be reacted to a growing chain of monomer units. In one particular example, the terminal 5'-hydroxyl group is caused to react with a deoxyribonucleoside-3'-O-(N,N-diisopropylamino)phosphoramidite protected at the 5'-position with dimethoxytrityl or the like. The 5' protecting group is removed after the coupling reaction, and the procedure is repeated with additional protected nucleotides until synthesis of the desired polynucleotide is complete. For a more detailed discussion of the chemistry involved in the above synthetic approaches, see, for example, U.S. Pat. No. 5,436,327 at column 2, line 34, to column 4, line 36, which is incorporated herein by reference in its entirety.

In general, in the above synthetic steps involving monomer addition such as, for example, the phosphoramidite method, there are certain repetitive steps such as washing the surface of the support prior to or after a reaction, oxidation of substances such as oxidation of a phosphite group to a phosphate group, removal of protecting groups, blocking of sites to prevent reaction at such site, capping of sites that did not react with a phosphoramidite reagent, deblocking, and so forth. In addition, under certain circumstances other reactions may be carried out in a flow cell such as, for example, phosphoramidite monomer addition, modified phosphoramidite addition, other monomer additions, addition of a polymer chain to a surface for linking to monomers, and so forth.

For in situ fabrication methods, multiple different reagent droplets are deposited by pulse jet or other means at a given target location in order to form the final feature (hence a probe of the feature is synthesized on the array substrate). The in situ fabrication methods include those described in U.S. Pat. No. 5,449,754 for synthesizing peptide arrays, and in U.S. Pat. No. 6,180,351 and WO 98/41531 and the references cited therein for polynucleotides, and may also use pulse jets for depositing reagents. The in situ method for fabricating a polynucleotide array typically follows, at each of the multiple different addresses at which features are to be formed, the same conventional iterative sequence used in forming polynucleotides from nucleoside reagents on a support by means of known chemistry. This iterative sequence can be considered as multiple ones of the following attachment cycle at each feature to be formed: (a) coupling an activated selected nucleoside (a monomeric unit) through a phosphite linkage to a functionalized support in the first iteration, or a nucleoside bound to the substrate (i.e. the nucleoside-modified substrate) in subsequent iterations; (b) optionally, blocking unreacted hydroxyl groups on the substrate bound nucleoside (sometimes referenced as "capping"); (c) oxidizing the phosphite linkage of step (a) to form a phosphate linkage; and (d) removing the protecting group ("deprotection") from the now substrate bound nucleoside coupled in step (a), to generate a reactive site for the next cycle of these steps. The coupling can be performed by depositing drops of an activator and phosphoramidite at the specific desired feature locations for the array. Capping, oxidation and deprotection can be accomplished by treating the entire substrate ("flooding") with a layer of the appropriate reagent. The functionalized support (in the first cycle) or deprotected coupled nucleoside (in subsequent cycles) provides a substrate bound moiety with a linking group for forming the phosphite linkage with a next nucleoside to be coupled in step (a). Final deprotection of nucleoside bases can be accomplished using alkaline conditions such as ammonium hydroxide, in another flooding procedure in a known manner. Conventionally, a single pulse jet or other dispenser is assigned to deposit a single monomeric unit.

The foregoing chemistry of the synthesis of polynucleotides is described in detail, for example, in Caruthers, Science 230: 281-285, 1985; Itakura, et al., Ann. Rev. Biochem. 53: 323-356; Hunkapillar, et al., Nature 310: 105-110, 1984; and in "Synthesis of Oligonucleotide Derivatives in Design and Targeted Reaction of Oligonucleotide Derivatives", CRC Press, Boca Raton, Fla., pages 100 et seq., U.S. Pat. Nos. 4,458,066, 4,500,707, 5,153,319, 5,869,643 and European patent application, EP 0294196, and elsewhere. The phosphoramidite and phosphite triester approaches are most broadly used, but other approaches include the phosphodiester approach, the phosphotriester approach and the H-phosphonate approach. The substrates are typically functionalized to bond to the first deposited monomer. Suitable techniques for functionalizing substrates with such linking moieties are described, for example, in Southern, E. M., Maskos, U. and Elder, J. K., Genomics, 13, 1007-1017, 1992.

In the case of array fabrication, different monomers and activator may be deposited at different addresses on the substrate during any one cycle so that the different features of the completed array will have different desired biopolymer sequences. One or more intermediate further steps may be required in each cycle, such as the conventional oxidation, capping and washing steps in the case of in situ fabrication of polynucleotide arrays (again, these steps may be performed in flooding procedure).

In the methods of the subject invention, the 5' functional group generating step (that includes oxidizing, optionally capping and deblocking (described in greater detail below)) occurs in a flow cell in many embodiments. As summarized above, the flow cell allows fluids to be passed through the chamber where the support is disposed. The support is mounted in the chamber in or on a holder. The housing usually further comprises at least one fluid inlet and at least one fluid outlet for flowing fluids into and through the chamber in which the support is mounted. In one approach, the fluid outlet may be used to vent the interior of the reaction chamber for introduction and removal of fluid by means of the inlet. On the other hand, fluids may be introduced into the reaction chamber by means of the inlet with the outlet serving as a vent and fluids may be removed from the reaction chamber by means of the outlet with the inlet serving as a vent.

The dimensions of the housing chamber of the employed flow cell may vary and are dependent on the dimensions of the support that is to be placed therein. In certain embodiments, the support may be one on which a single array of chemical compounds is synthesized. In this regard the support is usually about 1.5 to about 5 inches in length and about 0.5 to about 3 inches in width. The support is usually about 0.1 to about 5 mm, more usually, about 0.5 to about 2 mm, in thickness. A standard size microscope slide is usually about 3 inches in length and 1 inch in width. Alternatively, multiple arrays of chemical compounds may be synthesized on the support, which is then diced, i.e., cut, into single array supports. In this alternative approach the support is usually about 5 to about 8 inches in length and about 5 to about 8 inches in width so that the support may be diced into multiple single array supports having the aforementioned dimensions. The thickness of the support is the same as that described above. In a specific embodiment by way of illustration and not limitation, a wafer that is 6^{5/8} inches by 6 inches is employed and diced into one inch by 3 inch slides.

Representative flow cells that may be employed in certain embodiments are about 6.5 inches wide by about 6 inches tall in the plane of the flow cell. More generally these dimensions can range from the size of an array about 1 cm square to about 1 meter square. The gap width in representative embodiments of flow cells that may be employed in the invention ranges from about 1 µm to about 500 µm, and in certain embodiments can range from about 1-10 µm to about 10 mm.

Flow cell devices employed in array fabrication which are suitable for use with the subject invention are further described in U.S. Published Patent Application Nos. 20030003222; 20030003504; 20030112022; 200030228422; 200030232123; and 20030232140; the disclosures of which are herin incorporated by refeence.

The housing of the flow cell is generally constructed to permit access into the chamber therein. The flow cell may have an opening that is sealable to fluid transfer after the support is placed therein. Such seals may comprise a flexible material that is sufficiently flexible or compressible to form a fluid tight seal that can be maintained under increased pressures encountered in the use of the device. The flexible member may be, for example, rubber, flexible plastic, flexible resins, and the like and combinations thereof. In any event the flexible material should be substantially inert with respect to the fluids introduced into the device and must not interfere with the reactions that occur within the device. The flexible member is usually a gasket and may be in any shape such as, for example, circular, oval, rectangular, and the like. Preferably, the flexible member is in the form of an O-ring.

Alternatively, the housing of the flow cell may be conveniently constructed in two parts, which may be referred to generally as top and bottom elements. These two elements are sealably engaged during synthetic steps and are separable at other times to permit the support to be placed into and removed from the chamber of the flow cell. Generally, the top element is adapted to be moved with respect to the bottom element although other situations are contemplated herein. Movement of the top element with respect to the bottom element is achieved by means of, for example, pistons, and so forth. The movement is controlled electronically by means that are conventional in the art. In another approach a reagent chamber is formed in situ from a support and a sealing member. The inlet of the flow cell is usually in fluid communication with an element that controls the flow of fluid into the flow cell such as, for example, a manifold, a valve, and the like or combinations thereof. This element in turn is in fluid communication with one or more fluid reagent dispensing stations. In this way different fluid reagents for one step in the synthesis of the chemical compound may be introduced sequentially into the flow cell.

The inlet of the flow cell is usually in fluid communication with an element that controls the flow of fluid into the flow cell such as, for example, a manifold, a valve, and the like or combinations thereof. This element in turn is in fluid communication with one or more fluid reagent dispensing stations. In this way different fluid reagents for one step in the synthesis of the chemical compound may be introduced sequentially into the flow cell.

In one embodiment the fluid dispensing stations are affixed to a base plate or main platform to which the flow cells are mounted. Any fluid dispensing station may be employed that dispenses fluids such as water, aqueous media, organic solvents, ionic liquids and the like. The fluid dispensing station may comprises a pump for moving fluid and may also comprise a valve assembly and a manifold as well as a means for delivering predetermined quantities of fluid to the flow cell. The fluids may be dispensed by pumping from the dispensing station. In this regard any standard pumping technique for pumping fluids may be employed in the present apparatus. For example, pumping may be by means of a peristaltic pump, a pressurized fluid bed, a positive displacement pump, e.g., a syringe pump, and the like.

After the reagent in introduced into the flow cell, the reagent is held in contact with the support for a time and under conditions sufficient for the particular step to be completed. The time periods and conditions are dependent on the nature of the reagent and the nature of the particular step of the procedure. For example, the time periods and conditions may be different for a washing procedure rather than an oxidizing reaction or a deblocking reaction. In general, the time periods and conditions for the procedures conducted in the flow cells are well-known in the art and will not be repeated here.

Methods of producing nucleic acid arrays using an *in situ* nucleic acid synthesis protocol are provided, where the *in situ* nucleic acid synthesis protocol includes a plurality of cycles, each of which includes: (I) a monomer attachment step; and (II) a 5' functional group generation step. A feature of the subject methods is that the 5' functional group generation step is performed by sequentially flowing a plurality of different fluids, e.g., oxidizing, deblocking and washing fluids, across the surface of a substrate, e.g., in a flow cell, in a manner such that the surface is not contacted with with a triple phase interface line while the liquid phase contains an active reagent. Also provided are the arrays produced using the subject methods, as well as methods for use of the arrays and kits that include the same.

Before the subject invention is described further, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an" and "the" include plural reference unless the context clearly dictates otherwise. Unless defined otherwise, all technical and scientific terms used herein have the same meaning as commonly understood to one of ordinary skill in the art to which this invention belongs. Although any methods, devices and materials similar or equivalent to those described herein can be used in the practice or testing of the invention, the preferred methods, devices and materials are now described.

Where a range of values is provided, it is understood that each intervening value, to the tenth of the unit of the lower limit unless the context clearly dictates otherwise, between the upper and lower limit of that range, and any other stated or intervening value in that stated range, is encompassed within the invention. The upper and lower limits of these smaller ranges may independently be included in the smaller ranges, and are also encompassed within the invention, subject to any specifically excluded limit in the stated range. Where the stated range includes one or both of the limits, ranges excluding either or both of those included limits are also included in the invention.

All publications mentioned herein are incorporated herein by reference for the purpose of describing and disclosing the invention components which are described in the publications which might be used in connection with the presently described invention.

As summarized above, the subject invention provides methods of producing nucleic acid arrays, as well as the arrays produced thereby and various applications of using the same. In further describing the subject invention, the subject methods are described first in greater detail, followed by a more in-depth review of the arrays produced by the subject methods, representative applications in which the arrays find use, and kits for use in practicing the subject methods.

### METHODS

As summarized above, the subject invention provides methods of producing nucleic acid arrays. More specifically, the subject invention provides methods of producing nucleic acid arrays by *in situ* synthesis of two or more distinct nucleic acids on the surface of a solid support. The *in situ* synthesis protocol employed in the subject invention can be viewed as an iterative process that includes two or more cycles, where each cycle includes the following steps: (I) a monomer attachment step in which a blocked nucleoside monomer is covalently bonded to two or more distinct locations, e.g., at least a first and second location, of a functional group, e.g., hydroxyl, amino, etc., displaying surface of a solid support; and (II) an internucleotide linkage stabilization and 5' functional group generation step in which the phosphite triester linkage is oxidized and functional groups are generated at the blocked ends of the resultant attached blocked nucleotides by removal of the blocking groups for addition of subsequent nucleoside monomers.

In certain embodiments of interest, each cycle includes the following steps: (I) a monomer attachment step in which a 5'OH blocked nucleoside monomer is covalently bonded to two or more distinct locations, e.g., at least a first and second location, of a hydroxyl functional group displaying surface of a solid support, e.g., a nascent planar surface of a solid support displaying hydroxyl functional groups or a surface displaying intermediate nucleic acids having 5'OH groups; and (II) an internucleotide linkage stabilization and 5'OH generation step in which the phosphite triester linkage is oxidized and hydroxyl groups are generated at the 5' ends of the resultant attached blocked nucleotides by removal of the blocking groups for addition of subsequent nucleoside monomers, where this step includes oxidizing and deblocking substeps, as well as optionally a capping substep. Each of these cycle steps is now described separately in greater detail in terms of these particular embodiments. However, the scope of the invention is not so limited-the invention being described in terms of these particular representative embodiments for ease of description only.

### Monomer Attachment Step

In the monomer attachment step of each cycle, one or more different 5'OH blocked nucleoside monomers is contacted with one or more different locations of a substrate surface that displays hydroxyl functional groups, such that the nucleoside monomers become covalently bound to the surface, e.g., via a nucleophilic substitution reaction between the an activated (e.g., protonated) phosphoramidite moiety of the blocked nucleoside monomer and the surface displayed hydroxyl functionality. The surface displayed hydroxyl functionality may be on the surface of a nascent substrate, or may be at the 5' end of a growing nucleic acid, depending on the particular point in the synthesis protocol. For example, at the beginning of a particular synthesis protocol, the surface displayed hydroxyl functional groups are immediately on the surface of a solid support or substrate. In contrast, following one or more cycles of a given synthesis protocol, the surface displayed functional groups are present at the 5' ends of growing nucleic acids which, in turn, are covalently bonded to the surface of the solid support.

As such, at the beginning of any array synthesis protocol, the first step is to provide a substrate having a surface that displays hydroxyl functional groups, where the hydroxyl functional groups are employed in the covalent attachment of the growing nucleic acid ligands to the substrate surface during synthesis. The substrate may be any convenient substrate that finds use in biopolymeric arrays. In general, the substrate may be rigid or flexible. The substrates may be fabricated from a variety of materials. In certain embodiments, e.g., where one is interested in the production of nucleic acid arrays for use in research and related applications, the materials from which the substrate may be fabricated may exhibit a low level of non-specific binding during hybridization events. In many situations, it is of interest to employ a material that is transparent to visible and/or UV light. Specific materials of interest include: silicon; glass; plastics, e.g., polytetrafluoroethylene, polypropylene, polystyrene, polycarbonate, and blends thereof, and the like; metals, e.g. gold, platinum, and the like; etc. The surface may be modified with one or more different layers of compounds that serve to modify the properties of the surface in a desirable manner. Such modification layers, when present, will generally range in thickness from a monomolecular thickness to about 1 mm, usually from a monomolecular thickness to about 0.1 mm and more usually from a monomolecular thickness to about 0.001 mm. Modification layers of interest include: inorganic and organic layers such as metals, metal oxides, conformal silica or glass coatings, polymers, small organic molecules and the like. Polymeric layers of interest include layers of: peptides, proteins, polynucleic acids or mimetics thereof, e.g. peptide nucleic acids and the like; polysaccharides, phospholipids, polyurethanes, polyesters, polycarbonates, polyureas, polyamides, polyethyleneamines, polyarylene sulfides, polysiloxanes, polyimides, polyacetates, and the like, where the polymers may be hetero- or homopolymeric, and may or may not have separate functional moieties attached thereto, e.g. conjugated. The particular surface chemistry will be dictated by the specific process to be used in polymer synthesis, as described in greater detail infra. However, as mentioned above, the substrate that is initially employed has a surface that displays hydroxyl functional groups.

As mentioned above, nucleic acid arrays are produced according to the subject invention by synthesizing nucleic acid polymers using conventional phosphoramidite solid phase nucleic acid synthesis chemistry where the solid support is a substrate as described above. Phosphoramidite based chemical synthesis of nucleic acids is well known to those of skill in the art, being reviewed above and in U.S. Patent No. 4,415,732, the disclosure of the latter being herein incorporated by reference.

To produce nucleic acid arrays according to the subject methods, a substrate surface as described above having the appropriate surface groups, e.g., -OH groups, present on its surface, is obtained. Since the synthesis protocol must be carried out under anhydrous conditions, all reactions are carried out in a non-aqueous, typically organic solvent layer on the substrate surface, where the solvent layer is acetonitrile in many embodiments.

Next, the first residues of each nucleic acid to be synthesized on the array are covalently attached to the substrate surface via reaction with the surface bound -OH groups. Depending on whether the first nucleotide residue of each nucleic acid to be synthesized on the array is the same or different, different protocols for this step may be followed. Where each of the nucleic acids to be synthesized on the substrate surface have the same initial nucleotide at the 3' end, the entire surface of the substrate is contacted with the blocked, activated nucleoside under conditions sufficient for coupling of the activated nucleoside to the reactive groups, e.g. -OH groups, present on the substrate surface to occur. In these embodiments, the entire surface of the array may be contacted with the fluid composition containing the activated nucleoside using any convenient protocol, such as flooding the surface of the substrate with the activated nucleoside solution, immersing the substrate in the solution of activated nucleoside, etc. The fluid composition is typically a fluid composition of the blocked nucleoside in an organic solvent, e.g., acetonitrile, where the fluid composition typically includes an activating agent, e.g., tetrazole, benzoimidazolium triflate ("BZT"), S-ethyl tetrazole, and dicyanoimidazole, etc.

Alternatively, where the initial residue of the various nucleic acids differs among the nucleic acids, one or more sites on the substrate surface are individually contacted with fluid compositions of the appropriate blocked, activated nucleoside. In this latter embodiment, any convenient protocol for selectively contacting a particular site, region or cell of a substrate surface with a fluid composition of the activated nucleoside may be employed. Of particular interest in many embodiments is the use of pulse-jet deposition protocols, such as those described in U.S. Patent No. 6,171,797; 6,180,351; 6,232,072; 6,242,266; 6, 300,137; and 6,323,043; as well as U.S. Patent Application Serial No. 09/302,898 filed April 30, 1999; the disclosures of which documents, particularly with respect to their teaching of in situ array synthesis via pulse-jet deposition protocols, are herein incorporated by reference. In these embodiments, two or more different fluid compositions of activated, blocked nucleosides, which fluid compositions differ from each other in terms of the activated nucleoside present therein, are each pulse-jetted onto one or more distinct locations of the surface, where the locations are dictated by the sequence of the desired nucleic acid at each location.

The activated nucleoside monomers employed in this attachment step of each cycle of the subject synthesis methods are blocked at their 5'-OH functionalities (ends) with an acid labile blocking group. By acid labile blocking group is meant that the group is cleaved in the presence of an acid to yield a 5'-OH functionality. In many embodiments, the acid labile blocking group is DMT, as described above.

The above step of the subject protocols results in a "blocked monomer attached substrate" where the surface is characterized by the presence of blocked monomers, e.g., DMT blocked nucleoside monomers, covalently attached to the surface of a solid support, either directly if the blocked monomers are the first residues of to be synthesized surface bound nucleic acid ligands, or through a growing nucleic acid ligands, i.e., where blocked monomers are at the end of growing nucleic acid chains. This resultant "blocked monomer attached substrate" is then subjected to the next step of the subject synthesis cycle, i.e., the 5'OH generation step.

### Generation of Functionalities

As summarized above, following covalent attachment of activated, blocked nucleoside monomers to one or more locations of the substrate surface, functional, e.g., 5'OH hydroxyl, moieties are then generated on the surface so that the synthesis cycle can be repeated with a new round of activated, blocked nucleoside monomers. This generation step includes (in representative embodiments) the following substeps: (a) oxidation; (b) optional capping; and (c) deblocking.

A feature of the subject methods is that each of these substeps is accomplished by contacting the entire surface of the substrate with an appropriate fluid, i.e., an oxidation fluid, a capping fluid or a deblocking fluid, a wash fluid, etc. Contact of the entire surface is achieved in the subject methods by flooding the surface with the appropriate fluid, where a flow cell approach is employed in representative embodiments, such that the entire substrate is contacted with a volume of the appropriate liquid, e.g., by flowing a volume of the appropriate liquid over the surface of the substrate in an appropriate container or chamber, e.g., flow cell. As such, in representative embodiments, performance of each substep includes flowing an adequate volume of the appropriate fluid over the substrate surface so that the entire surface of the substrate is contacted with the fluid.

In representative embodiments, wash reagent is first allowed to pass into and out of the flow cell. Next, oxidizing agent is introduced into the flow cell. Following an additional wash step, the support is then subjected to a deblocking step. In this step, a deblocking reagent for removing a protecting group is flooded over the substrate surface. Next, wash fluid contained in a fluid dispensing station that is in fluid communication with the flow cell may be flooded over the substrate. Optionally, the substrate surface may be contacted with a capping fluid that includes a capping agent, where the surface may be contacted with a capping fluid at one or more times, e.g., prior to oxidation, prior to deblocking, etc. Following the above steps, the support may be transported from the flow cell to the printing chamber where the next monomer addition is carried out and the above repetitive synthetic steps are conducted as discussed above.

The amount of the reagents employed in each of the above steps in the method of the present invention is dependent on the nature of the reagents, solubility of the reagents, reactivity of the reagents, availability of the reagents, purity of the reagents, and so forth. Such amounts should be readily apparent to those skilled in the art in view of the disclosure herein. Usually, stoichiometric amounts are employed, but excess of one reagent over the other may be used where circumstances dictate. Typically, the amounts of the reagents are those necessary to achieve the overall synthesis of the chemical compound in accordance with the present invention. The time period for conducting the present method is dependent upon the specific reaction and reagents being utilized and the chemical compound being synthesized.

When moving the support or substrate between the printing and flow cell stations, the support may be transported by a transfer element such as a robotic arm, and so forth. In one embodiment a transfer robot is mounted on a main platform of an apparatus for carrying out the above syntheses. The transfer robot may comprise a base, an arm that is movably mounted on the base, and an element for grasping the support during transport that is attached to the arm. The element for grasping the support may be, for example, movable finger-like projections, and the like. In use, the robotic arm is activated so that the support is grasped by the above-mentioned element. The arm of the robot is moved so that the support is delivered to the flow cell.

In view of the above, the functional group generation step may be viewed as a process in which the substrate surface is sequentially contacted or flooded with a plurality of two or more different fluids, for example three or more fluids, including four or more fluids, such as oxidizing fluid, wash fluid, deblocking fluid, and optionally capping fluid.

As summarized above, a feature of the subject invention is that during the functional generation step, the substrate surface is not exposed to or contacted with a triple phase interface line. As such, the substrate surface (at least that which is to be occupied by the polymeric features in the final array product) is not simultaneously contaced with a gas and liquid. In representative embodiments, the substrate surface is not contacted with a gas during the functional group generation step. In other words, the substrate surface is not subjected to a triple interface phase line of gas, solid and liquid. To prevent exposure of the substrate surface to a gas, in representative embodiments of the subject methods, excess fluid or solution employed in a given substep is removed from the substrate surface prior to performing the next substep by purging or displacing the fluid from the surface with the immediately subsequent fluid in the series of fluids that is to be contacted with the surface. In other words, in sequentially contacting the surface with the plurality of fluids, any given previous fluid in the sequential plurality of fluids is removed from the surface by displacing that fluid with the immediately subsequent fluid. For example, where a given 5' functional group generation step requires sequential contact of a surface with the following liquids in the following order: (1) cap liquid; (2) wash liquid; (3) oxidizing liquid; (4) cap liquid; (5) wash liquid; (6) deblock liquid; (7) wash liquid; each prior liquid in the sequence of liquids is displaced or purged from the surface with the immediately following liquid, such that the first cap liquid is purged by the wash liquid; the second wash liquid is purged by the oxidizing liquid; the third oxidizing liquid is purged by the cap liquid; the fourth cap liquid is purged by the fifth wash liquid; the fifth wash liquid is purged by the sixth deblock liquid; and the sixth deblock liquid is purged by the seventh wash liquid. For convenience, an immediately subsequent liquid that is employed to purge the prior liquid from the surface is referred to in the following paragraphs as the "purging fluid or liquid."

As summarized above, a feature of the subject invention is that in sequentially contacting the surface of the substrate with a plurality of fluids during the functional group generation step, any prior fluid in the sequence or series is removed from the surface by purging or displacing it from the surface with the immediately following fluid (e.g., liquid), referred to conveniently as the purging fluid or liquid.

In certain embodiments, the preceding or prior fluid is displaced from the surface by flowing the purging fluid across the surface in a manner that produces a defined or stratified interface or front between the purging fluid and the preceding fluid, which defined interface is maintained as it moves across the substrate surface and the prior fluid is concomitantly displaced therefrom. This technique uses pressure gradient driven stratification. In representative embodiments of the subject invention, the pressure gradient is brought about by gravity through orientation of the flow cell at least partially vertical. By at least partially vertical is meant that the angle between the plane of the flow cell and the horizontal plane of the environment, e.g., room, in which the flow cell is present is at least about 5°, at least about 10 °such as at least about 15°, including at least about 30°, e.g., at least about 45°, 60°, 75° and up to 90 °. In yet other embodiments, centrifugal acceleration may be employed to generate the desired pressure gradients.

The rate at which the purging fluid is flowed across the surface of the substrate to displace the preceding or prior fluid of the sequence is chosen to maintain a substantially stratified front or interface between the purging and prior fluids as the front progresses across the substrate. As such, the flow rate of the purging fluid is selected so as to achieve minimal mixing of the purging and preceding fluids as the preceding fluid is displaced or purged from the substrate surface. The chosen rate at which the purging fluid is flowed across the surface of the substrate in the flow cell may be based on consideration of the following principles of fluid flow through a flow cell. As is known by those of skill in the art, the characteristics of fluid flow within the flowcell are determined by the Reynolds number (Re), where Re= ρ (density)*U(velocity of fluid flow)* gapwidth/viscosity. The Re for the flow cells employed in certain embodiments of the subject invention is or about o(100) and is strictly laminar, even in the presence of unstable density fronts. As such, consideration may be given to the characteristics of the laminar flow with respect to the boundary layer of material that remains close to the substrate or wafer surface as the purging fluid is introduced into the flowcell. As the purging fluid passes over the substrate surface, a thin layer of the prior fluid will be left on the substrate surface that must be diffused from the surface into the bulk flow of the purging fluid. As is known to those of skill in the art, the characteristics of this flow are determined by the Peclet number (Pe) where Pe = U * b / D where U is the centerline speed, b is the gap width and D is the molecular diffusivity of the active deblocking agent in the wash solvent. For very high Pe the convective bulk flow dominates and there is little time for material to diffuse into the bulk flow, which can be undesirable in the present invention. At low Pe, molecular diffusion allows the purging fluid and prior fluid to interpenetrate via diffusion thus allowing the surface to be substantially cleansed of prior fluid (e.g., by the mechanism known to those of skill in the art as the Taylor dispersion). In representative embodiments, the rate at which the purging fluid is flowed across the substrate surface may range from about 1 cm/s to about 20cm/s.

As indicated above, the purging fluid is, in representative embodiments, a fluid of different density relative to the prior fluid that is being displaced or purged. A measure of the density difference is given by the Atwood number (A) which is equal to (ρ1 - ρ2)/(ρ1 + ρ2), where ρ1 is the density of the fluid on the bottom and ρ2 is the density of the fluid superposed on top of the lower fluid. In representative embodiments, the purging fluid is chosen such that the density difference (A) between the purging fluid and the prior fluid or solution that is being displaced is greater than 0, and in certain embodiments ranges from about 0.001 to about 0.5, including from about 0.01 to about 0.2.

As summarized above, the above 5' functional group generation step is performed using a flow cell in many representative embodiments. Accordingly, for example, after addition of a nucleoside monomer, such as depositing the reagent using a pulse-jet method, the support is placed into a chamber of a flow cell, which is typically a housing having a reaction cavity or chamber disposed therein. The flow cell allows fluids to be passed through the chamber where the support is disposed. The support may be mounted in the chamber in or on a holder. The housing usually further comprises at least one fluid inlet and at least one fluid outlet for flowing fluids into and through the chamber in which the support is mounted. In one approach, fluids may be introduced into the reaction chamber by means of the inlet with the outlet serving as a vent and fluids may be removed from the reaction chamber by means of the outlet with the inlet serving as a vent. As such, all of the fluids in the plurality of fluids contacted with the surface are contacted with the surface in a "first-in-first-out" manner.

The inlet of the flow cell may be in fluid communication with an element that controls the flow of fluid into the flow cell such as, for example, a manifold, a valve, and the like or combinations thereof. This element in turn is in fluid communication with one or more fluid reagent dispensing stations. In this way different fluid reagents for one step in the synthesis of the chemical compound may be introduced sequentially into the flow cell. These reagents may be, for example, wash fluids, oxidizing agents, reducing agents, blocking or protecting agents, unblocking (deblocking) or deprotecting agents, and so forth, as indicated above and described in greater detail below. Any reagent that is normally a solid reagent may be converted to a fluid reagent by dissolution in a suitable solvent, which may be a protic solvent or an aprotic solvent. The solvent may be an aqueous medium that is solely water or may contain a buffer, or may contain from about 0.01 to about 80 or more volume percent of a cosolvent such as an organic solvent as mentioned above. The solvent may, in certain embodiments, be an ionic liquid.

Following the 5'-generation step, summarized above, the remaining fluid, e.g., wash fluid, may be removed from the surface, e.g., by draining, and the surface dried.

In performing the above-described substeps, while the order of oxidation and blocking may be reversed, the deblocking step is typically performed following capping/oxidation. As such, the capping/oxidation steps are described together first, followed by a description of the deblocking step. It should be noted that capping before oxidation also prevents formation of branched DNA, while capping after oxidation also removes moisture introduced by the oxidation. In some protocols, capping is done before and after oxidation. As such, capping may be performed before oxidation, after oxidation, or both before and after oxidation.

It should be noted that the following descriptions of deblocking, oxidizing, capping and wash fluids are merely representative, and that other types of fluids may be employed in a given protocol, e.g., a combined oxidizing/deblocking fluid, such as that described in Published United States Application No. 20020058802, the disclosure of which is herein incorporated by reference.

### Oxidation

Oxidation results in the conversion of phosphite triesters present on the substrate surface following coupling to phosphotriesters. Oxidation is accomplished by contacting the surface with an oxidizing solution, as described above, which solution includes a suitable oxidating agent. Various oxidizing agents may be employed, where represenative oxidizing agents include, but are not limited to: organic peroxides, oxaziridines, iodine, sulfur etc. The oxidizing agent is typically present in a fluid solvent, where the fluid solvent may include one or more cosolvents, where the solvent components may be organic solvents, aqueous solvents, ionic liquids, etc. A representative oxidizing agent of interest is I₂/H₂O/Pyridine/THF. Following contact of the surface with the oxidizing solution, excess is removed as described above.

### Optional Capping

In addition, unreacted hydroxyl groups may be (though not necessarily) capped, e.g., using any convenient capping agent, as is known in the art. This optional capping is accomplished by contacting the surface with an capping solution, as described above, which solution includes a suitable capping agent, such as a solution of acetic anhydride, pyridine or 2,6-lutidine (2,6-dimethylpyridine), and tetrahydrofuran ("THF"); a solution of 1-methyl-imidazole in THF; etc. Following contact of the surface with the oxidizing solution, excess oxidizing solution is removed as described above.

### Deblocking

The next substep in the subject methods is the deblocking step, where acid labile protecting groups present at the 5' ends of the growing nucleic acid molecules on the substrate are removed to provide free 5' OH moieties, e.g., for attachment of subsequent monomers, etc. In this deblocking step (which may also be referred to as a deprotecting step as results in removal of the protecting blocking groups), the entire substrate surface is contacted with a deblocking or deprotecting agent, typically in a flow cell, as described above. The substrate surface is incubated for a sufficient period of time under appropriate conditions for all available protecting groups to be cleaved from the nucleotides that they are protecting.

In certain representive embodiments, the deblocking solution includes an acid present in an organic solvent that has a low vapor pressure. The vapor pressure of the organic solvent that is employed in the deblocking solution is typically at least substantially the same as toluene, by which is meant that the vapor pressure is not more than about 350% and usually not more than about 150% of the vapor pressure of toluene at a given set of temperature/pressure conditions. In certain embodiments, the organic solvent is one that has a vapor pressure that is less than about 13 KPa, usually less than about 8 KPa and more usually less than about 5 KPa at standard temperature and pressure conditions i.e., STP conditions (0°C; 1 ATM). A variety of organic solvents are of interest, where such solvents include, but are not limited to: toluene, xylene (o, m, p), ethylbenzene, perfluoro-n-heptane, perfluoro decalin, chlorobenzene, 1,2 dichloroethane, 1,1,2 trichloroethane, 1,1,2,2 tetrachloroethane, pentachloroethane, and the like; where in many embodiments, the organic solvent that is employed is toluene. The acid deblocking agent employed in the deblocking solution may vary, where representative acids of interest include, but are not limited to: acetic acids, e.g.,acetic acid, mono acetic acid, dichloroacetic acid, trichloroacetic acid, monofluoroacetic acid, difluoroacetic acid, trifluoroacetic acid, and the like. The amount of acid in the solution is sufficient to remove blocking groups, and typically ranges between about 0.1 and 20%, more typically ranges between 1 and 3%, as is known in the art.

Contact of the substrate surface with a deblocking solution results in removal of the protecting groups from the blocked substrate bound residues. As such, this step results in the deprotection of the nucleotide residues on the substrate surface. Following deprotection, the deblocking solution is removed from the surface of the substrate.

Removal of the deblocking agent according to the subject methods results in a substrate surface in which the nucleotide residues are deprotected. In others words, removal of the deblocking agent results in the production of an array of nucleotide residues stably associated with the substrate surface, where the nucleotide residues on the array surface have 5' -OH groups available for reaction with an activated nucleotide in subsequent cycles.

### Washing

In typical representative embodiments, the surface of the substrate is washed between one or more of the above described capping, oxidation and deblocking steps. Any convenient wash fluid may be employed in these one or more wash steps. In certain embodiments, the wash fluid may be a low viscosity fluid. In these embodiments, the viscosity of the wash fluid typically does not exceed about 1.2, and in certain embodiments does not exceed about 0.6, such as about 0.4 cP (as measured at 25°C). The non-dimensional capillary number of the flow should be in the range of from about 10⁻² to about 10⁻⁶. The capillary number Ca is defined as Ca = (µ×U)/σ, where µ is the viscosity, U is the linear speed and σ is the surface tension. This number provides a range within which the substrate or wafer drag-out speed can be adjusted to account for the particular fluid properties. However, while Ca serves as a coarse guide for controlling mechanical aspects of the flow, other subtleties such as the evaporation rate and fluid adherence to the substrate manifested in the disjoining pressure influence the motion of the contact line. Such embodiments are employed where it is desired for the any liquid film remaining on the surface of the substrate following fluid removal to evaporate rapidly.

In certain embodiments, the wash fluid is an organic solvent or an ionic liquid. In certain embodiments, solvents of from 1 to about 6, more usually from 1 to about 4, carbon atoms, including alcohols such as methanol, ethanol, propanol, etc., ethers such as tetrahydrofuran, ethyl ether, propyl ether, etc., acetonitrile, dimethylformamide, dimethylsulfoxide, and the like, may be employed. Specific organic solvents of interest include, but are not limited to: acetonitrile, acetone, methanol, ethanol and the like.

The above steps of: (a) monomer attachment; and (b) functional group regeneration, e.g., 5'OH hydroxyl regeneration, are repeated a number of times with additional monomers, e.g., nucleotides until each of the desired polymers, e.g., nucleic acids on the substrate surface are produced. By choosing which sites are contacted with which activated nucleotides, e.g. A, G, C & T, an array having polymers of desired sequence and spatial location is readily achieved.

As such, the above cycles of monomer attachment and functional (e.g., hydroxyl) moiety regeneration result in the production of an array of desired polymers, e.g., nucleic acids. The resultant arrays can be employed in a variety of different applications, as described in greater detail below.

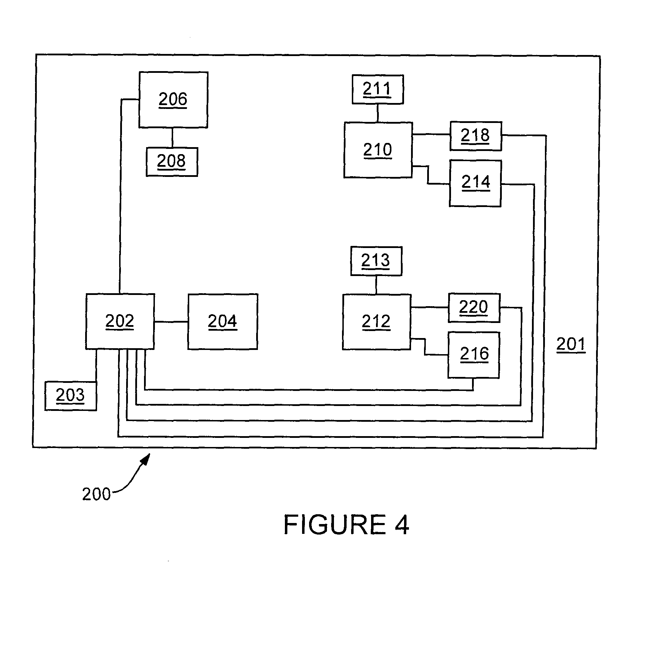

The above method steps may be carried out manually or with a suitable automated device, where in many embodiments a suitable automated device is employed. Of particular interest is an automated device that can automatically transfer a substrate from an activated monomer deposition location, i.e., a "writer station" to a surface processing station where the above steps of capping, oxidation and deblocking are carried out, e.g., a wet chemical processing station in which the substrate surface is automatically contacted with the appropriate fluids in a sequential fashion. A representative automated manufacturing device that is adapted to perform the subject methods is depicted in Figure 4 and described in greater detail below.

As indicated above, the above description describing use of 5'OH functional groups, acid labile blocking groups, such as DMT and the use of an acid deblocking agent, are merely representative. Various modifications may be made and still fall within the scope of the invention. For example, other functional groups may be employed, e.g., amine functional groups. In yet other embodiments, base labile blocking groups may be employed, where such groups and the use thereof are described in U.S. Patent No. 6,222,030; the dislcosure of which is herein incorporated by reference. In these latter types of embodiments, the acid deblocking agent described above is replaced with a base deblocking agent. In yet other embodiments, the "direction" of synthesis may be reversed, such that the synthesized nucleic acids are attached to the substrate at their 5' ends and one generats 3' functional groups in the deblocking/deprotecting step.

The subject invention has been described above in terms of fabrication of nucleic acids arrays. While the above description has been provided in terms of nucleic acid array production protocols for ease and clarity of description, the scope of the invention is not so limited, but instead extends to the fabrication of any type of array structure, particularly biopolymeric array structure, including, but not limited to polypeptide arrays, in addition to the above described nucleic acid arrays. The subject invention is particularly useful for the fabrication of arrays using a protocol that includes a deblocking step, such as the representative deblocking step described above, where a blocking group is removed at some point during an iterative synthesis process.

It should also be noted that the above description of the invention is described in terms of purging a first fluid with a second fluid. In many representative embodiments, the first and second fluids are liquids. However, in certain embodiments, the first and second fluids may be gases, such that a first gas is displaced by a second gas. In these latter embodiments, the method is characterized not in that it is one in which a gas is not contacted with the surface of the substrate during functional group regeneration, but that a liquid is not contacted with the surface of the substrate during functional group regeneration, such that the substrate surface is not contacted with a triple phase interface line or intersection, as noted above.

### ARRAY MANUFACTURING DEVICES

One embodiment of an apparatus that comprises one or more flow cell assemblies in accordance with the present invention is depicted in FIG. 4 in schematic form. Apparatus 200 comprises platform 201 on which the components of the apparatus are mounted. Apparatus 200 comprises main computer 202, with which various components of the apparatus are in communication. Video display 203 is in communication with computer 202. Apparatus 200 further comprises print chamber 204, which is controlled by main computer 202. The nature of print chamber 204 depends on the nature of the printing technique employed to add monomers to a growing polymer chain. Such printing techniques include, by way of illustration and not limitation, pulse-jet deposition printing, and so forth. Transfer robot 206 is also controlled by main computer 202 and comprises a robot arm 208 that moves a support to be printed from print chamber 204 to either first flow cell assembly 210 or second flow cell assembly 212. In one embodiment robot arm 208 introduces a support into print chamber 204 horizontally for printing on a surface of the support and introduces the support into a flow cell vertically. First flow cell assembly 210 is in communication with program logic controller 214 (which corresponds to controller 106 of FIG. 1), which is controlled by main computer 202, and second flow cell 212 is in communication with program logic controller 216, which is also controlled by main computer 202. First flow cell 210 assembly is in communication with fluid dispensing station 211 and flow sensor and level indicator 218, which are controlled by main computer 202, and second flow cell assembly 212 is in communication with fluid dispensing station 213 and flow sensor and level indicator 220, which are also controlled by main computer 202.

The apparatus of the invention further includes appropriate electrical and mechanical architecture and electrical connections, wiring and devices such as timers, clocks, and so forth for operating the various elements of the apparatus. Such architecture is familiar to those skilled in the art and will not be discussed in more detail herein.

The methods in accordance with the present invention may be carried out under computer control, that is, with the aid of a computer. For example, an IBM® compatible personal computer (PC) may be utilized. The computer is driven by software specific to the methods described herein. Computer hardware capable of assisting in the operation of the methods in accordance with the present invention involves in certain embodiments a system with at least the following specifications: Pentium® processor or better with a clock speed of at least 100 MHz, at least 32 megabytes of random access memory (RAM) and at least 80 megabytes of virtual memory, running under either the Windows 95 or Windows NT 4.0 operating system (or successor thereof). Software that may be used to carry out the methods may be, for example, Microsoft Excel or Microsoft Access, suitably extended via user-written functions and templates, and linked when necessary to stand-alone programs. Examples of software or computer programs used in assisting in conducting the present methods may be written, preferably, in Visual BASIC, FORTRAN and C⁺⁺. It should be understood that the above computer information and the software used herein are by way of example and not limitation. The present methods may be adapted to other computers and software. Other languages that may be used include, for example, PASCAL, PERL or assembly language.

A computer program may be utilized to carry out the above method steps. The computer program provides for controlling the valves of the flow assemblies to introduce reagents into the flow cells, vent the flow cells, and so forth. The computer program further may provide for moving the support to and from a station for monomer addition at a predetermined point in the aforementioned method.

Another aspect of the present invention is a computer program product comprising a computer readable storage medium having a computer program stored thereon which, when loaded into a computer, performs the aforementioned method.

In representative embodiments, the methods are coded onto a computer-readable medium in the form of "programming", where the term "computer readable medium" as used herein refers to any storage or transmission medium that participates in providing instructions and/or data to a computer for execution and/or processing. Examples of storage media include floppy disks, magnetic tape, CD-ROM, a hard disk drive, a ROM or integrated circuit, a magneto-optical disk, or a computer readable card such as a PCMCIA card and the like, whether or not such devices are internal or external to the computer. A file containing information may be "stored" on computer readable medium, where "storing" means recording information such that it is accessible and retrievable at a later date by a computer.

With respect to computer readable media, "permanent memory" refers to memory that is permanent. Permanent memory is not erased by termination of the electrical supply to a computer or processor. Computer hard-drive ROM (i.e. ROM not used as virtual memory), CD-ROM, floppy disk and DVD are all examples of permanent memory. Random Access Memory (RAM) is an example of non-permanent memory. A file in permanent memory may be editable and rewritable.

A "computer-based system" refers to the hardware means, software means, and data storage means used to analyze the information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means may comprise any manufacture comprising a recording of the present information as described above, or a memory access means that can access such a manufacture.

A "processor" references any hardware and/or software combination which will perform the functions required of it. For example, any processor herein may be a programmable digital microprocessor such as available in the form of a electronic controller, mainframe, server or personal computer (desktop or portable). Where the processor is programmable, suitable programming can be communicated from a remote location to the processor, or previously saved in a computer program product (such as a portable or fixed computer readable storage medium, whether magnetic, optical or solid state device based). For example, a magnetic medium or optical disk may carry the programming, and can be read by a suitable reader communicating with each processor at its corresponding station.

In certainembodiments, the processor will be in operable linkage, i.e., part of or networked to, the aforementioned device, and capable of directing its activities.

### ARRAYS

Also provided by the subject invention are arrays of nucleic acids produced according to the subject methods, as described above. The subject arrays include at least two distinct nucleic acids that differ by monomeric sequence immobilized on, e.g., covalently to, different and known locations on the substrate surface. In certain embodiments, each distinct nucleic acid sequence of the array is typically present as a composition of multiple copies of the polymer on the substrate surface, e.g., as a spot on the surface of the substrate. The number of distinct nucleic acid sequences, and hence spots or similar structures, present on the array may vary, but is generally at least 2, usually at least 5 and more usually at least 10, where the number of different spots on the array may be as a high as 50, 100, 500, 1000, 10,000 or higher, depending on the intended use of the array. The spots of distinct polymers present on the array surface are generally present as a pattern, where the pattern may be in the form of organized rows and columns of spots, e.g., a grid of spots, across the substrate surface, a series of curvilinear rows across the substrate surface, e.g., a series of concentric circles or semi-circles of spots, and the like. The density of spots present on the array surface may vary, but will generally be at least about 10 and usually at least about 100 spots/cm², where the density may be as high as 10⁶ or higher, but will generally not exceed about 10⁵ spots/cm². In other embodiments, the polymeric sequences are not arranged in the form of distinct spots, but may be positioned on the surface such that there is substantially no space separating one polymer sequence/feature from another. As indicated above, the arrays are arrays of nucleic acids, including oligonucleotides, polynucleotides, DNAs, RNAs, synthetic mimetics thereof, and the like.

A feature of the subject arrays, which feature results from the protocol employed to manufacture the arrays, is that each probe location of the arrays is highly uniform in terms of probe composition, since substantially no depurination side reactions occur during the array processing, if any. As such, the proportion of full-length sequence within each feature is higher as compared to arrays produced using analogous protocols but not the subject deblock removal step, as described herein (e.g., at least about 5-fold higher, often at least about 10-fold higher, such as at least about 25-, 50- or 75-fold higher), and the length distribution within each feature is less skewed towards shorter sequences. As a result, background noise and non-selective signal are reduced in the hybridization signal.

### UTILITY

The subject arrays find use in a variety of different applications, where such applications are generally analyte detection applications in which the presence of a particular analyte in a given sample is detected at least qualitatively, if not quantitatively. Protocols for carrying out such assays are well known to those of skill in the art and need not be described in great detail here. Generally, the sample suspected of comprising the analyte of interest is contacted with an array produced according to the subject methods under conditions sufficient for the analyte to bind to its respective binding pair member that is present on the array. Thus, if the analyte of interest is present in the sample, it binds to the array at the site of its complementary binding member and a complex is formed on the array surface. The presence of this binding complex on the array surface is then detected, e.g. through use of a signal production system, e.g. an isotopic or fluorescent label present on the analyte, etc. The presence of the analyte in the sample is then deduced from the detection of binding complexes on the substrate surface.

Specific analyte detection applications of interest include hybridization assays in which the nucleic acid arrays of the subject invention are employed. In these assays, a sample of target nucleic acids is first prepared, where preparation may include labeling of the target nucleic acids with a label, e.g. a member of signal producing system. Following sample preparation, the sample is contacted with the array under hybridization conditions, whereby complexes are formed between target nucleic acids that are complementary to probe sequences attached to the array surface. The presence of hybridized complexes is then detected. Specific hybridization assays of interest which may be practiced using the subject arrays include: gene discovery assays, differential gene expression analysis assays; nucleic acid sequencing assays, and the like. Patents and patent applications describing methods of using arrays in various applications include: 5,143,854; 5,288,644; 5,324,633; 5,432,049; 5,470,710; 5,492,806; 5,503,980; 5,510,270; 5,525,464; 5,547,839; 5,580,732; 5,661,028; 5,800,992; the disclosures of which are herein incorporated by reference.

In certain embodiments, the subject methods include a step of transmitting data from at least one of the detecting and deriving steps, as described above, to a remote location. By "remote location" is meant a location other than the location at which the array is present and hybridization occur. For example, a remote location could be another location (e.g. office, lab, etc.) in the same city, another location in a different city, another location in a different state, another location in a different country, etc. As such, when one item is indicated as being "remote" from another, what is meant is that the two items are at least in different buildings, and may be at least one mile, ten miles, or at least one hundred miles apart. "Communicating" information means transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data. The data may be transmitted to the remote location for further evaluation and/or use. Any convenient telecommunications means may be employed for transmitting the data, e.g., facsimile, modem, internet, etc.

As such, in using an array made by the method of the present invention, the array will typically be exposed to a sample (for example, a fluorescently labeled analyte, e.g., protein containing sample) and the array then read. Reading of the array may be accomplished by illuminating the array and reading the location and intensity of resulting fluorescence at each feature of the array to detect any binding complexes on the surface of the array. For example, a scanner may be used for this purpose which is similar to the AGILENT MICROARRAY SCANNER scanner available from Agilent Technologies, Palo Alto, CA. Other suitable apparatus and methods are described in U.S. patent applications: Serial No. 09/846125 "Reading Multi-Featured Arrays" by Dorsel et al.; and Serial No. 09/430214 "Interrogating Multi-Featured Arrays" by Dorsel et al. As previously mentioned, these references are incorporated herein by reference. However, arrays may be read by any other method or apparatus than the foregoing, with other reading methods including other optical techniques (for example, detecting chemiluminescent or electroluminescent labels) or electrical techniques (where each feature is provided with an electrode to detect hybridization at that feature in a manner disclosed in US 6,221,583 and elsewhere). Results from the reading may be raw results (such as fluorescence intensity readings for each feature in one or more color channels) or may be processed results such as obtained by rejecting a reading for a feature which is below a predetermined threshold and/or forming conclusions based on the pattern read from the array (such as whether or not a particular target sequence may have been present in the sample or an organism from which a sample was obtained exhibits a particular condition). The results of the reading (processed or not) may be forwarded (such as by communication) to a remote location if desired, and received there for further use (such as further processing).

### KITS

Finally, kits for use in analyte detection assays are provided. The subject kits at least include the arrays of the subject invention. The kits may further include one or more additional components necessary for carrying out an analyte detection assay, such as sample preparation reagents, buffers, labels, and the like. As such, the kits may include one or more containers such as vials or bottles, with each container containing a separate component for the assay, and reagents for carrying out an array assay such as a nucleic acid hybridization assay or the like. The kits may also include a denaturation reagent for denaturing the analyte, buffers such as hybridization buffers, wash mediums, enzyme substrates, reagents for generating a labeled target sample such as a labeled target nucleic acid sample, negative and positive controls and written instructions for using the subject array assay devices for carrying out an array based assay. The instructions may be printed on a substrate, such as paper or plastic, etc. As such, the instructions may be present in the kits as a package insert, in the labeling of the container of the kit or components thereof (i.e., associated with the packaging or sub-packaging) etc. In other embodiments, the instructions are present as an electronic storage data file present on a suitable computer readable storage medium, e.g., CD-ROM, diskette.

It is evident from the above results and discussion that an important new protocol for preparing nucleic acid (as well as other types) of arrays is provided by the subject invention. Specifically, the subject methods provide for automated protocols of *in situ* synthesis of nucleic acid arrays with greatly reduced depurination side reactions resulting from the deblocking step, resulting in in situ production of arrays with highly uniform features. Accordingly, the subject invention represents a significant contribution to the art.

All publications and patent applications cited in this specification are herein incorporated by reference as if each individual publication or patent application were specifically and individually indicated to be incorporated by reference. The citation of any publication is for its disclosure prior to the filing date and should not be construed as an admission that the present invention is not entitled to antedate such publication by virtue of prior invention.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

## Claims

1. A method of producing an array of at least two different polymeric ligands covalently bonded to a surface of a substrate, said method comprising:
(a) contacting blocked monomers to at least a first location and a second location of a substrate (110) having a surface displaying functional groups under conditions sufficient for said blocked monomers to covalently bond to said surface in said first and second locations to produce a substrate surface displaying covalently bound blocked monomers;
(b) removing blocking groups of said blocked monomers in a functional group generation step in a manner such that said surface is not exposed to a triple phase interface line of a gas, solid and liquid; and
(c) reiterating steps (a) and (b) at least once to produce said array of at least two polymeric ligands.

2. The method according to Claim 1, wherein said functional group generation step (b) comprises sequentially contacting at least a portion of said surface with a plurality of different liquids.

3. The method according to Claim 2, wherein said plurality of different liquids includes at least an oxidizing fluid and a deblocking fluid.

4. The method according to Claim 3, wherein said plurality of different liquids further includes a wash liquid.

5. The method according to any of Claims 2 to 4, wherein any two sequentially applied liquids of said plurality have a different density.

6. The method according to Claim 5, wherein any two sequentially applied liquids of said plurality have a different density (A) of greater than zero.

7. The method according to any of Claims 2 to 6, wherein said plurality of liquids is sequentially contacted with said surface by displacing a previous liquid of said plurality with an immediately subsequent liquid.

8. The method according to Claim 7, wherein said displacing comprises flowing said immediately subsequent liquid across said surface in a manner sufficient to produce a stratified liquid interface between said immediately subsequent and previous liquids that moves across said surface.

9. An apparatus (200) for synthesizing an array of biopolymers on the surface of a support, said apparatus comprising:
(a) a reaction chamber (210, 212);
(b) a mechanism (206) for moving a support to and from said reaction chamber;
(c) a controller (202) for controlling the movement of said mechanism of step (b);
(d) one or more fluid dispensing stations (211, 213) in fluid communication with said reaction chamber;
(e) a controller (202) for controlling said mechanism of (d) in a manner according to the method of claims 1 to 8;
(f) a mechanism for activating said fluid dispensing stations to independently dispense reagents to the surface of a support, said mechanism being cooperative with said mechanism of (d); and
(g) a controller (202) for controlling said mechanism of (e), and 9f) one or more additional chambers for conducting reactions that form part of said synthesis.

10. A computer-readable medium comprising:
programming for controlling the apparatus of claim 9 according to the method of any of Claims 1 to 8.
